(19) Europäisches Patentamt
European Patent Office
Office européen des brevets

(11) EP 3 357 475 A1

(12) EUROPEAN PATENT APPLICATION

(43) Date of publication:
08.08.2018 Bulletin 2018/32

(51) Int Cl.:
A61H 31/00 (2006.01)   A61N 1/39 (2006.01)
A61B 5/00 (2006.01)   A61B 5/0205 (2006.01)
A61B 5/021 (2006.01)   A61B 5/024 (2006.01)
G06F 19/00 (2018.01)

(21) Application number: 17154735.9

(22) Date of filing: 06.02.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Koninklijke Philips N.V.
5656 AE Eindhoven (NL)

(72) Inventors:
• DELLIMORE, Kiran Hamilton J.
5656 AE Eindhoven (NL)
• MUEHLSTEFF, Jens
5656 AE Eindhoven (NL)

(74) Representative: de Haan, Poul Erik
Philips International B.V.
Philips Intellectual Property & Standards
High Tech Campus 5
5656 AE Eindhoven (NL)

(54) HARVESTING ENERGY FROM PERFORMANCE OF CARDIOPULMONARY RESUSCITATION

(57) The present disclosure is directed to methods and apparatus for harvesting energy from performance of CPR, and utilizing that energy for various purposes. In some embodiments, a system may include: a portable component (102) that is adapted for placement on an outer surface of a patient's body such that chest compressions applied during performance of CPR on the patient causes corresponding motion of the portable component; an electrical generator (104) coupled to the portable component and adapted to convert the motion of the portable component into electrical energy; motion sensor(s) (108) adapted to detect aspect(s) of the motion of the portable component; and a memory component (112) communicatively coupled with the motion sensor(s), wherein the memory component is adapted to be powered by at least some of the electrical energy to at least temporarily store data point(s) associated with the aspect(s) of the motion of the portable component.

100

PORTABLE HOUSING 102

| MEMORY 112 | WIRELESS 116 | ELECTRICAL GENERATOR 104 |

LOGIC 110

114

ENERGY STORAGE 106

| CLOCK 120 | SENSOR(S) 108 | OUTPUT 118 |

Fig. 1

EP 3 357 475 A1

**Description**

FIELD OF THE INVENTION

The present invention is directed generally to health care. More particularly, but not exclusively, various methods and apparatus disclosed herein relate to apparatus and techniques for harvesting energy from performance of cardiopulmonary resuscitation ("CPR"), and utilizing that energy for various purposes.

BACKGROUND OF THE INVENTION

Lay rescuers that are called upon to perform CPR in a medical emergency may lack sufficient training and/or confidence in their abilities, due to inexperience, passage of time since training, etc. Accordingly, there is a significant need for lay rescuers to receive reassurance that they are correctly performing CPR, or an alert indicating that they are performing CPR incorrectly. Various devices exist that provide feedback and guidance during CPR. However, a major limitation of these devices is that they are not ubiquitously available during a health emergency and even when available may not be reliable since they require batteries, which may expire. Self-powering CPR mannequins (or "dummies") exist that are used to train would-be rescuers to perform CPR by providing detecting chest compressions performed on the mannequin and providing feedback. However, these devices are bulky (they include a fake body), complex, expensive, and only available at CPR training facilities. They are not available or helpful in real-life situations in which CPR is required. Accordingly, there is a need to address these problems by providing a reliable means for powering a CPR feedback device for lay rescuers which is ubiquitously available and reliable.

SUMMARY OF THE INVENTION

The present disclosure is directed to methods and apparatus for harvesting energy from performance of CPR, and utilizing that energy for various purposes. For example, in various embodiments, motion associated with applied chest compressions and/or decompressions maybe converted to electrical energy. As used herein, the term "compressions" refers to both compressions (pushing into the patient's chest) and decompressions (pulling up from the patient's chest) unless otherwise indicated. The electrical energy may be used to power various electronic components to monitor CPR performance and provide feedback and/or guidance.

Generally, in one aspect, a system for harvesting and using electrical energy during performance of CPR may include: a portable component (housing) that is adapted for placement on an outer surface of a patient's body such that chest compressions applied during performance of CPR on the patient causes corresponding motion of the portable component; an electrical generator coupled to the portable component and adapted to convert the motion of the portable component into electrical energy; one or more motion sensors adapted to detect one or more aspects of the motion of the portable component; and a memory component communicatively coupled with the one or more sensors, wherein the memory component is powered by at least some of the electrical energy to at least temporarily store one or more data points associated with the one or more aspects of the motion of the portable component.

In various embodiments, the system may further include one or more output devices adapted to be powered by the electrical energy to provide CPR quality feedback generated based on the one or more aspects of the motion of the portable component detected by the one or more motion sensors. In various embodiments, the electrical generator may take the form of a linear alternator adapted to convert the motion of the portable component into the electrical energy.

In various embodiments, the system may further include one or more wireless components adapted to be powered by the electrical energy to wirelessly transmit one or more of the data points associated with performance of the CPR to a remote computing device. In various embodiments, one or more of the motion sensors may be coupled to the portable component and are adapted to be powered by the electrical energy. In various embodiments, one or more of the motion sensors may be integral with a smart watch worn by an individual that performs the CPR. In various embodiments, the one or more of the motion sensors that are integral with the smart watch may be adapted to wirelessly transmit one or more of the data points to the portable component.

In various embodiments, the portable component may include a smart phone or smart glasses. In various embodiments, the portable component may include a planar housing that is adapted to be placed on or near the patient's chest. In various embodiments, the one or more motion sensors may include an accelerometer or a force sensor. In various versions, the system may further include comprising logic adapted to analyze a signal from the accelerometer or force sensor and compare the signal to one or more rules associated with performance of CPR. In various embodiments, the electrical generator may take the form of a piezo-ceramic component.

In another aspect, a method may include: placing a portable component on an outer surface of a body of a patient; applying chest compressions to the patient during performance of CPR on the patient, wherein the chest compressions cause corresponding motion of the portable component; converting, by an electrical generator coupled to the portable component, the motion of the portable component into electrical energy; detecting, by one or more motion sensors, one or more aspects of the motion of the portable component; and powering a wireless component with the electrical energy to wirelessly transmit, to a remote computing device, one or more data points associated with the one or more aspects of the motion of the portable component.

In yet another aspect, an apparatus for providing feedback during performance of CPR may include: a portable housing that is adapted for placement on an outer surface of a body of a patient such that chest compressions applied during performance of CPR on the patient causes corresponding motion of the portable housing; an electrical generator contained within the portable housing and adapted to convert the motion of the portable housing into electrical energy; one or more motion sensors contained within the portable housing, wherein the one or more motion sensors are powered by the electrical energy and adapted to detect one or more aspects of the motion of the portable housing; and one or more wireless components contained in the portable housing and communicatively coupled with the one or more motion sensors. In various embodiments, the one or more wireless components may be powered by at least some of the electrical energy to wirelessly transmit one or more data points associated with the one or more aspects of the motion of the portable housing to a remote computing device. In various embodiments, transmission of the one or more data points may cause the remote computing device to provide output indicative of the feedback.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.

Fig. 1 illustrates schematically an example portable component configured with selected aspects of the present disclosure, in accordance with various embodiments.
Fig. 2 depicts an example environment in which disclosed techniques maybe practiced.
Fig. 3 schematically depicts guidelines for performing CPR, which may be performed using a portable component configured with selected aspects of the present disclosure.
Fig. 4 depicts an example method of performing various techniques described herein, in accordance with various embodiments.

DETAILED DESCRIPTION OF THE EMBODIMENTS

Lay rescuers that are called upon to perform CPR in a medical emergency may lack sufficient training and/or confidence in their abilities, due to inexperience, passage of time since training, etc. Accordingly, there is a significant need for lay rescuers to receive reassurance that they are correctly performing CPR, or an alert indicating that they are performing CPR incorrectly. Various devices exist that provide feedback and guidance during CPR. However, a major limitation of these devices is that they are not ubiquitously available during a health emergency and even when available may not be reliable since they require batteries, which may expire. Accordingly, techniques are described herein for harvesting and/or storing energy from chest compression during CPR and then discharging this energy in a controlled manner to power various components, such as memory, sensors which record CPR quality data, wireless communication components, output devices that provide feedback to the lay rescuer, and so forth.

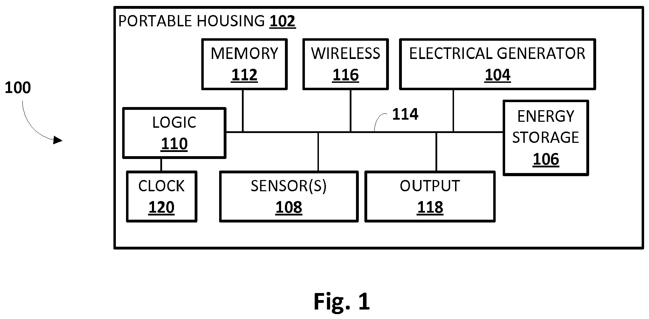

Referring to Fig. 1, an example CPR-energy-harvesting system 100 configured with selected aspects of the present disclosure is depicted schematically. CPR-energy-harvesting system 100 may include a portable housing 102 that may be constructed with various materials (e.g., polymers, metals, textiles, etc.) and that may have various shapes, such as a planar disk (or "puck") shape, cuboid, square, round, etc. In some embodiments, portable housing 102 may take other forms such as a textile glove or a band that is worn by a rescuer during performance of CPR. In some embodiments, portable housing 102 may be adapted for placement on an outer surface of patient's body (not depicted in Fig. 1) such as on the patient's chest during performance of CPR. Consequently, chest compressions applied during performance of CPR on the patient may cause corresponding motion (i.e., kinetic energy) of portable housing 102.

In various embodiments, portable housing 102 may include an electrical generator 104 that is adapted to convert the motion (i.e.,, kinetic energy) of portable housing 102 into electrical energy, e.g., for temporary storage (e.g., as potential energy) in energy storage component 106 and/or for immediate use. Energy storage component 106 may take various forms, such as a rechargeable battery, one or more capacitors or super capacitors, and so forth. In some embodiments, electrical generator 104 may take the form of a linear or rotary alternator that converts energy from motion associated with chest compressions into electrical energy, e.g., according to Faraday's Law. For example, a linear alternator may include a magnet that moves back and forth (e.g., oscillates) inside of or otherwise adjacent to a multiturn coil. Movement of the magnet during compression and/or decompression of the patient's chest may induce current in the coil.

In other embodiments, electrical generator 104 may take the form of piezo-ceramic component (e.g., a plate, card, etc.), which may be constructed, for instance, using lead zirconate titanate (a.k.a. "PZT"). Motion of and/or force applied to portable housing 102 may bend such a piezo-ceramic component to generate the electrical energy. In some embodiments, the harvested energy may be immediately discharged, e.g., via a sound-acoustic modulator, visible light communication (e.g., 802.15.7), or an antenna, to wirelessly transmit measured CPR quality data to a nearby remote computing device, such as a smart watch worn by the rescuer and/or another nearby individual.

Portable housing 102 may also contain one or more sensors 108, such as motion sensors, adapted to detect one or more aspects of the motion of and/or force applied to portable housing 102. For example, in various embodiments, sensors 108 may include one or more of an accelerometer, gyroscope and/or a force sensor. In some embodiments, other sensors may be provided in addition to motion sensors, such as vital sign sensors (e.g., photoplethysmogram or "PPG" sensors to detect heart rate), thermometers, microphones, and so forth. In various embodiments, a signal produced by the one or more sensors 108 may be provided to a logic 110. Logic 110 may take various forms, such as one or more microprocessors that execute instructions stored in a memory component 112, an application-specific integrated circuit ("ASIC"), a field-programmable gate array ("FPGA"), and so forth.

In various embodiments, memory component 112 and other components depicted in Fig. 1 maybe communicatively coupled with logic 110 and/or each other via one or more buses 114. Memory component 112 may take various forms that may or may not be considered "low power" because only a minimal amount of electrical energy is required to read from, and/or write to the memory component. In some embodiments, memory component 112 maybe non-volatile, and may include one or more of the following: random access memory ("RAM"), programmable ready-only memory ("PROM"), erasable PROM ("EPROM"), electrically erasable PROM ("EEPROM"), flash memory (NAND or NOR), Ferroelectric RAM ("FeRAM"), Programmable metallization cell ("CBRAM"), parallel random-access machine ("PRAM"), Silicon-Oxide-Nitride-Oxide-Silicon ("SONOS"), Resistive random-access memory ("RRAM"), racetrack memory, Nano-RAM, and so forth.

In various embodiments, one or more components depicted in Fig. 1, such as sensors 108, memory component 112, one or more wireless components 116, one or more output devices 118, and/or logic 110 itself may be powered in whole or in part by electrical energy generated by electrical generator 104 (and in some instances at least temporarily stored in energy storage component 106). Accordingly, CPR-energy-harvesting system 100 may be used without requiring batteries (which may expire, malfunction, etc.), and may always be available in emergency situations.

In various embodiments, one or more sensors 108 may provide signals to logic 110 that are indicative of one or more aspects of the motion and/or force undergone by and/or imparted on portable housing 102 during performance of CPR on a patient. Logic 110 and/or memory component 112, powered at least in part by electrical energy harvested by electrical generator 104 from the same motion/force, may at least temporarily store one or more data points associated with the one or more aspects of the motion of/force on portable housing 102. Data points that may be provided by sensors such as accelerometers and/or force sensors may include, but are not limited to, compression rate or frequency, force applied, compression depth, and so forth. Other sensors may provide signals indicative of other data points to logic 110, such as patient response (e.g., pulse or heart rate detected by a PPG sensor or microphone), etc. For example, in some embodiments, electrical energy harvested by electrical generator 104 may be wirelessly transmitted to a nearby pulse detection device (e.g., PPG sensor) that maybe placed, for instance, at the carotid or forehead of the patient.

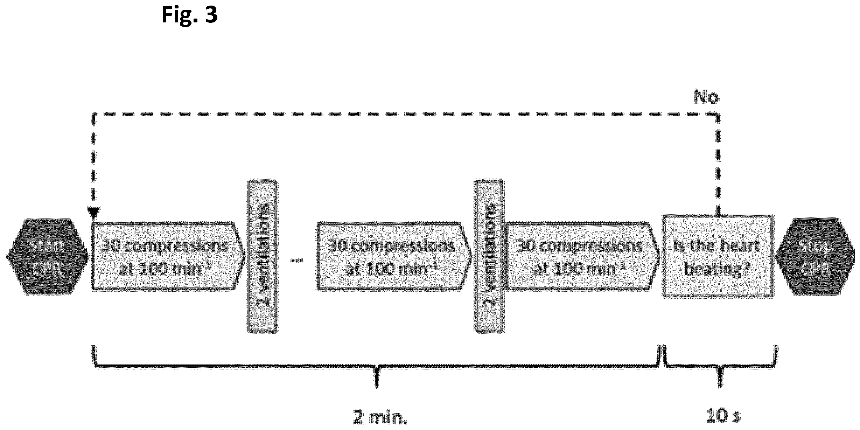

In some embodiments, the one or more data points stored in memory component 112 at least temporarily may be analyzed and/or used, e.g., by logic 110, to generate, e.g., at one or more output devices 118, audible, visual, and/or haptic feedback. CPR feedback may include various information relating to CPR performance quality, such as that compressions are too fast, too slow, too hard, too soft, too deep, not deep enough, or that it is time for a rescuer to apply ventilation (e.g., by breathing or otherwise pushing air into the patient's airway), to check for a pulse, and/or to hand over to another rescuer due to fatigue. In some embodiments, one or more output devices (e.g., light sources) may be used to convey the patient's pulse rate, including whether the patient has a pulse or not. In various embodiments, various signal processing algorithms and/or CPR rules maybe stored, e.g., in memory component 112, so that logic 110 can use these algorithms and/or rules to determine effectiveness of a rescuer's CPR performance. For example, in some embodiments, guidelines such as those depicted in Fig. 3 may be used to formulate an algorithm and/or rules that may be used by logic 110 to determine, based on signals received from one or more sensors 108, whether CPR is being performed correctly.

In some embodiments, output device 118 may include one or more light sources, such as one or more light-emitting diodes ("LEDs"), which may be energized to convey various information, such as CPR feedback or guidance.

In some embodiments, output device 118 may include a display device that may be configured to render various information, including CPR feedback or guidance. Such a display device may, in some instances, be a touchscreen or a low power e-ink screen, but this is not required. In other embodiments, output device 118 may include one or more speakers that are used to audibly convey CPR feedback. In yet other embodiments, particularly embodiments in which portable housing 102 is adapted to occupy a space held between the rescuer's hand and the patient's chest, output device 118 may include a haptic feedback mechanism that is configured to convey CPR feedback, e.g., using vibrations, etc. In various embodiments, logic 110 may include an internal clock that is used to cause one or more output devices 118 to provide the rescuer with a metronome or other similar timing feedback (audible, visual, and/or haptic) to aid in performance of CPR.

In some embodiments, one or more wireless components 116 may include a transmitter and/or a receiver (in some cases, a transceiver that transmits and receives) operably coupled with logic 110 (or if no logic is present, memory component 112). Wireless component 116 may be configured to communicate wirelessly with remote computing devices (not depicted in Fig. 1) using various low energy wireless technologies, such as Bluetooth low energy (e.g., Bluetooth 4.0), ANT, ANT+, infrared data association ("IrDA"), ZigBee, Z-Wave, low power Wi-Fi, passive Wi-Fi, near field communication ("NFC"), EnOcean radiofrequency transmission protocol, and so forth. In other embodiments, portable housing 102 may be connected with one or more remote computing devices using one or more wires, such as bit-serial cables, Universal Serial Bus ("USB"), etc.

In various embodiments, one or more data points, e.g., stored at least temporarily in memory component 112, may be transmitted by wireless component 116 to one or more remote computing devices, such as a mobile device (e.g., smart phone, laptop, tablet, wearable device) operated by a user (e.g., a patient and/or medical personnel). For example, a chest compression rate/force/depth and/or one or more detected vital signs may be transmitted to a mobile device (e.g., smart phone, smart watch, smart glasses, etc.) carried by the rescuer. In some embodiments, electrical energy harvested during the chest compressions (and undergone by portable housing 102) may be used to power wireless transmission of such data to one or more remote mobile computing devices.

In some embodiments, logic 110 may store the data point(s), e.g., in association with a timestamp generated by and received from a remote mobile computing device via wireless component 116. In other embodiments, logic 110 may store the data points in association with a timestamp generated locally, e.g., by logic 110 itself using a real time clock ("RTC") 120. In some embodiments, real time clock 120 may be powered locally, e.g., with a button cell battery (not depicted) installed on or in portable housing 102. Real time clock 120, which in some embodiments may be an integrated circuit (e.g., an ASIC) or other similar component, may be the only component powered by such a battery. Accordingly, because real time clocks are typically considered low power consumption devices, the button cell battery's stored electrical energy may last a relatively long time. Of course in other embodiments, real time clock 120 may be omitted, e.g., in favor of generating timestamps remotely from portable housing 102.

While not depicted in Fig. 1, in some embodiments, portable housing 102 may include a biasing mechanism such as a spring that is adapted to resist the force applied by the rescuer during chest compressions. In some instances, this added resistance may be selected so that the rescuer is still able to effectively perform CPR, but also so that more electrical energy can be harvested by electrical generator 104. Of course, in other embodiments, no such biasing mechanism may be employed.

Assume that an average compression force F applied by a rescuer during chest compression is 300 Newton's (N). Suppose further that such a force is provided over the 5.0-6.0 cm of sternal displacement d that is recommended by resuscitation guidelines, at a chest compression rate f of 100-200 compressions per minute ("cpm"), which is equivalent to about 1.67-2.0 Hz. The power output P that is generated can be calculated using an equation such as the following:

$$P = f \times F(d) = 1.67\text{-}2.0\text{Hz} \times 300\text{N} \times (5.0\text{-}6.0\text{cm}) = \pm 25.0\text{-}36.0 \text{ W} \qquad (1)$$

According, the total energy Etotal harvested in every two-minute period of chest compression during CPR may be calculated as follows:

$$E_{total} = P \times 120 \text{ seconds} = \pm 3.0\text{-}4.3 \text{ kJ} \qquad (2)$$

Assume 50% energy loss, and further assume that logic 110 and one or more sensors 108 (e.g., an accelerometer and a force sensor) each consume no more than 0.2W during data acquisition. Energy consumed during data acquisition over two minutes may be approximately 72J.

During feedback, assume output devices 118 such as LED lights consume no more than 0.1 W, logic 110 consumes 0.1 W, a speaker consumes 0.5W, storage of one or more data points in memory component 112 requires no more than 0.1 W, and that wireless data transmission by wireless component 116 also requires no more than 0.1 W. Energy consumed to provide feedback over two minutes may then be approximately 84J, energy consumed for data storage may be approximately 12J, and energy consumed during data transmission may be approximately 12J. Energy loss may be approximately 1.5kJ.

Thus, the net energy generated, Enet, may be calculated with the following equation:

$$Enet = \pm\, 3.0kJ - (72J + 84J + 12J + 12J - 1.5kJ) = \pm\, 1.3kJ \quad (3)$$

In some embodiments, outside power may be used instead for data transmission, reducing energy requirements further. For example, in some embodiments, NFC transmission may be initiated by a remote computing device to obtain data from memory 112 of portable housing 102 (in which case wireless component 116 may be a passive wireless transmitter). Additionally or alternatively, in some embodiments, wireless data transmission may not be performed continuously, and instead may be performed sporadically, e.g., during ten-second pauses between chest compression periods in which a pulse check is performed. Moreover, 300N for chest compression may be a conservative estimate, as it may not be uncommon for forces of up to 900N to be applied, which would yield far greater amounts of electrical energy.

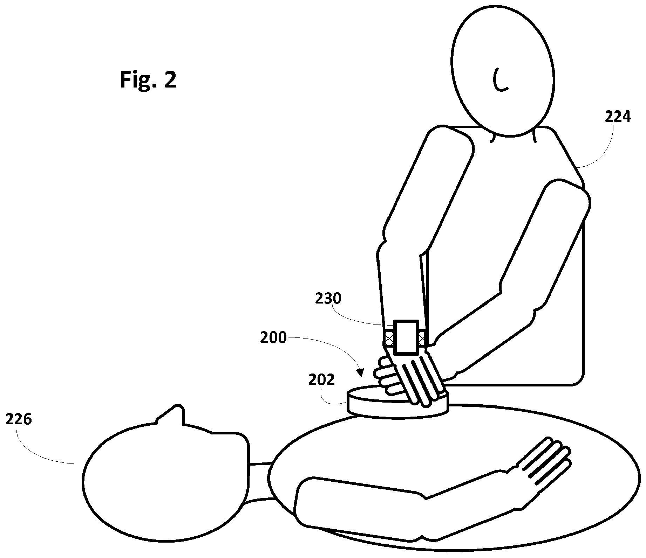

Fig. 2 schematically depicts an environment in which a system 200 configured with selected aspects of the present disclosure may be employed to aid a rescuer 224 performing CPR on a patient 226. In Fig. 2, system 200 may include a portable housing 202 configured with at least some of the aspects described above with respect to Fig. 1. In some embodiments, the embodiment depicted in Fig. 2 may be slightly different than that of Fig. 1 in that the one or more sensors, particularly the accelerometer and/or the force sensor, maybe incorporated into a portable computing device operated by rescuer 224, rather than within portable housing 202.

For example, rescuer 224 is depicted wearing a smart watch 230. Smart watch 230 may include, among other things, sensors such as an accelerometer and/or force sensor, which may effectively be "commandeered" by portable housing 202 to detect motion associated with performance of CPR by rescuer 224 on patient 226. In such embodiments, portable housing 202 may or may not necessarily include its own integral accelerometer and/or force sensor. A wireless component (116 in Fig. 1) of portable housing 202 may communicate wirelessly with smart watch 230 (e.g., using Bluetooth, ZigBee, NFC, EnOcean, Wi-Fi, modulated lighting, etc.) to obtain signals from the accelerometer and/or force sensor integral with smart watch 230. As noted above, portable housing 102 may harvest electrical energy to perform this wireless transmission from motion/force associated with the CPR. In some embodiments, smart watch 230 have a specialized "app" installed that facilitates cooperation (e.g., pairing) with portable housing 202. In some embodiments, portable housing 202 may itself store an installable version of the specialized app, and may make the app available for wireless download/installation directly from portable housing 202 by remote computing devices such as smart watch 230. That way, a lay rescuer who might not otherwise already have the app installed may receive the app automatically, e.g., when they attempt to perform CPR on patient 226. In some embodiments, such an app may not necessarily require deliberate user input to be launched at the remote computing device. For instance, the app maybe launched automatically by a command from the portable housing 202, and the app maybe given priority over other apps installed and running on the remote computing device.

In other embodiments in which rescuer 224 is not wearing smart watch 230, rescuer 224 may place a smart phone on the chest of patient 226, either between the rescuer's hands and the chest, or elsewhere on the patient's chest. An accelerometer and/or force sensor in the smart phone may be, as described above, "commandeered" by portable housing 202, as described above, to wireless provide data from its integral accelerometer and/or force sensor to portable housing 202.

In yet other embodiments, portable housing 202 may include its own internal sensors, and may merely communicate data indicative of readings of the sensors (e.g., accelerometer and/or force sensor readings) to the remote computing device (e.g., smart watch 230). In some embodiments, the remote computing device (e.g., smart watch 230) may store this date in association with one or more timestamps that, for instance, maybe generated at the remote computing device (e.g., if portable housing 202 does not include an internal real time clock). In some embodiments, portable housing 202 may or may not include its own integral output device(s), and may effectively "commandeer" one or more output devices of the remote computing device, such as a display, speaker, and/or haptic feedback mechanism integral with smart watch 230, to convey CPR feedback and/or guidance to rescuer 224. For example, portable housing 202 may transmit signals to smart watch 230 that causes smart watch 230 to vibrate in a timed manner that informs rescuer 224 as to whether they are performing CPR correctly, and/or provides haptic/audible output such as a metronome pulse to guide CPR chest compression performance by rescuer 224.

Fig. 3 depicts a flowchart of a CPR protocol recommended by European Resuscitation Counsel ("ERC"). As is shown, the rescuer (e.g., 224 in Fig. 2) should provide thirty chest compressions (which would include thirty corresponding decompressions) at a rate of one hundred compressions per minute (cpm). As noted above, a portable housing configured with selected aspects of the present disclosure maybe placed on a surface of the patient's body, such as the patient's chest, that is likely to undergo movement/force caused by CPR chest compressions performed by the rescuer.

Then, the rescuer is to provide two ventilations, e.g., by performing so-called "mouth-to-mouth" resuscitation or by using a hand-powered air pump (e.g., bag valve mask). The rescuer is to repeat these two sequences two more times, which should total approximately two minutes. Then, the rescuer should check to see if the heart is beating (e.g., which may include a ten-second pause). In some embodiments, the rescuer may take the patient's pulse manually, e.g., by feel (i.e., manual palpation). In other embodiments, a portable housing configured with selected aspects of the present disclosure may include a PPG sensor (which may or may not be powered by electrical energy harvested by electrical generator 104 during performance of CPR) that is adapted to detect the patient's pulse using a PPG signal. Or, in some embodiments, a rescuer carrying a smart phone with the above-described CPR app installed may utilize one or more sensors on the smart phone to detect the patient's pulse, e.g., by holding the smart phone on the patient's skin. If no heart beat is detected, then the rescuer may repeat the aforementioned sequence of steps.

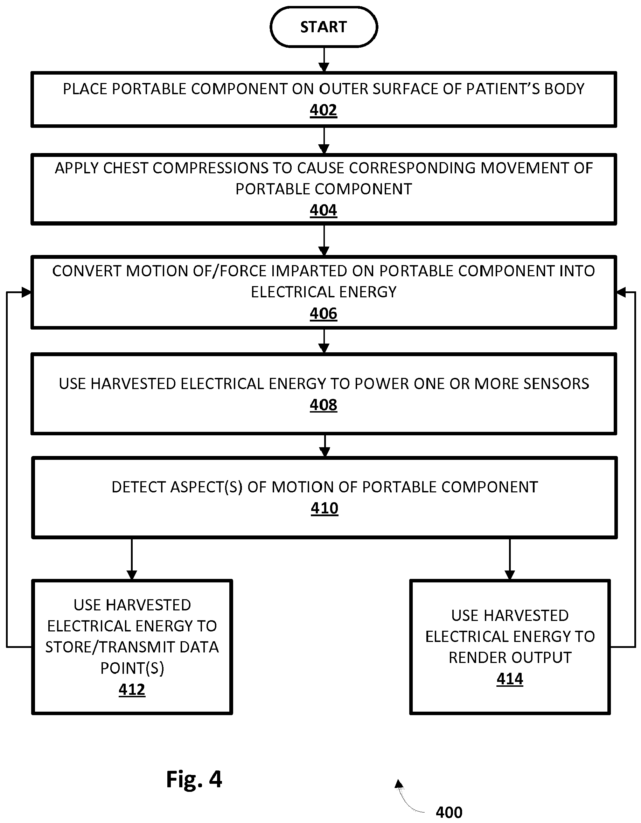

Fig. 4 depicts an example method 400 of performing various techniques described herein, in accordance with various embodiments. Although particular operations of method 400 are shown in a particular order, this is not meant to be limiting. In various embodiments, one or more operations may be added, omitted, and/or reordered.

At block 402, a portable component (e.g., 102, 202) maybe placed on an outer surface of a patient's body. In some embodiments the portable component may be placed underneath the rescuer's hands as they perform chest compressions. In other embodiments, the portable component may be placed elsewhere on the patient's body, such as elsewhere on the patient's chest, on the patient's stomach, etc., or even worn on or near the rescuer's hand, e.g., as a watch or glove.

At block 404, the rescuer may apply chest compressions to the patient during performance of CPR on the patient. As noted above, with the portable component placed on the patient's body, the chest compressions may cause corresponding motion/force of the portable component. At block 406, motion of/force applied to of the portable component, whether in its entirety or from portions of the portable component moving (e.g., bending) relative to other portions, may be converted into electrical energy. For example, a linear alternator may move up and down with the chest compressions, causing a magnet to move relative to a coil, thereby inducing current in the coil. Additionally or alternatively, the portable component may include a piezo-ceramic (e.g., PZT) planar portion that is deformed (bended) by the rescuer's chest compressions. Deformation of the piezo-ceramic planar portion may generate measureable piezoelectricity when its relatively static structure is deformed. This piezoelectricity may be harvested as electrical energy. Although not depicted in Fig. 4, in some embodiments, the harvested electrical energy may be stored at least temporarily in one or more energy storage components, such as a rechargeable battery and/or a capacitor or super capacitor.

At block 408, the electrical energy harvested at block 406 maybe used to power one or more sensors, such as one or more accelerometers, force sensors, etc. At block 410, one or more aspects of the motion undergone by the portable component, such as compression rate, compression depth, compress force, etc., may be detected by the one or more sensors that received power at block 408. And as noted above with respect to Fig. 2, in some embodiments, accelerometers and/or force sensors integral with remote computing devices may effectively be commandeered, in which case they may not necessarily be powered by the electrical energy harvested at block 406.

In some embodiments, at block 412, the electrical energy harvested at block 406 may be used to power a memory component and/or a wireless component to store and/or transmit (to one or more remote computing devices) one or more data points associated with the detected aspects of the motion undergone by the portable component. Additionally or alternatively, in some embodiments, at block 414, the electrical energy harvested at block 406 may be used to power one or more output devices integral with or otherwise coupled to the portable component to render audible/visual/haptic output that provides CPR quality feedback and/or guidance to the rescuer. For example, if the rescuer is applying chest compressions too frequently, or not frequently enough, audible, haptic, and/or visual output may be provided to notify the rescuer. If the rescuer is applying too much force, or too little force, audible, haptic, and/or visual output may be provided to notify the rescuer. In some embodiments, general guidance may be provided, e.g., in the form of an audible/visual/haptic pulse or metronome beat. From block 412 and/or block 414, method 400 may proceed back to block 406.

In another aspect of the invention an apparatus for providing feedback during performance of cardiopulmonary resuscitation ("CPR") is provided, said comprising: a portable housing that is adapted for placement on an outer surface of a body of a patient; an electrical generator contained within the portable housing and adapted to convert, into electrical energy, motion of or force imparted upon the portable housing as a result of chest compressions applied during performance of CPR on the patient; one or more motion sensors contained within the portable housing, wherein the one or more motion sensors are powered by the electrical energy and adapted to detect one or more aspects of the motion of the portable housing; one or more wireless components contained in the portable housing and communicatively coupled with the one or more motion sensors, wherein the one or more wireless components are powered by at least some of the electrical energy to wirelessly transmit one or more data points associated with the one or more aspects of the motion of the portable housing to a remote computing device, wherein transmission of the one or more data points causes the remote computing device to provide output indicative of the feedback.

In said apparatus the one or more motion sensors may include an accelerometer or a force sensor. Further, in said apparatus the electrical generator may comprise a linear alternator adapted to convert the motion of the portable component into the electrical energy or a piezo-ceramic component. The one or more motion sensors may include an accelerometer or a force sensor.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03. It should be understood that certain expressions and reference signs used in the claims pursuant to Rule 6.2(b) of the Patent Cooperation Treaty ("PCT") do not limit the scope.

**Claims**

1. A system (100) for harvesting and using electrical energy during performance of cardiopulmonary resuscitation ("CPR"), comprising:

   - a portable component (102) that is adapted for placement on an outer surface of a patient's body such that chest compressions applied during performance of CPR on the patient cause corresponding motion of the portable component;
   - an electrical generator (104) coupled to the portable component and adapted to convert the motion of the portable component into electrical energy;
   - one or more motion sensors (108) adapted to detect one or more aspects of the motion of the portable component; and
   - a memory component (112) communicatively coupled with the one or more motion sensors, wherein the memory component is adapted to be powered by at least some of the electrical energy to at least temporarily store one or more data points associated with the one or more aspects of the motion of the portable component.

2. The system of claim 1, further comprising one or more output devices (118) adapted to be powered by the electrical energy to provide CPR quality feedback generated based on the one or more aspects of the motion of the portable component detected by the one or more motion sensors.

3. The system of claim 1, wherein the electrical generator comprises a linear alternator adapted to convert the motion of the portable component into the electrical energy.

4. The system of claim 1, further comprising one or more wireless components (116) adapted to be powered by the electrical energy to wirelessly transmit one or more of the data points associated with performance of the CPR to a remote computing device.

5. The system of claim 1, wherein one or more of the motion sensors are coupled to the portable component and are adapted to be powered by the electrical energy.

6. The system of claim 1, wherein one or more of the motion sensors are integral with a smart watch worn (230) by an individual that performs the CPR.

7. The system of claim 6, wherein the one or more of the motion sensors that are integral with the smart watch are adapted to wirelessly transmit one or more of the data points to the portable component.

8. The system of claim 1, wherein the portable component comprises a smart phone or smart glasses.

9. The system of claim 1, wherein the portable component comprises a planar housing that is adapted to be placed on or near the patient's chest.

10. The system of claim 1, wherein the one or more motion sensors include an accelerometer or a force sensor.

11. The system of claim 10, further comprising logic adapted to analyze a signal from the accelerometer or force sensor and compare the signal to one or more rules associated with performance of CPR.

12. The system of claim 1, wherein the electrical generator comprises a piezo-ceramic component.

13. A method (400) comprising:

- placing (402) a portable component (102) on an outer surface of a body of a patient;
- applying (404) chest compressions to the patient during performance of CPR on the patient, wherein the chest compressions cause corresponding motion of the portable component;
- converting (406), by an electrical generator (104) coupled to the portable component, the motion of the portable component into electrical energy;
- detecting (410), by one or more motion sensors (108), one or more aspects of the motion of the portable component; and
- powering (412) a wireless component (116) with the electrical energy to wirelessly transmit, to a remote computing device, one or more data points associated with the one or more aspects of the motion of the portable component.

14. The method of claim 13, further comprising powering a memory component with the electrical energy to at least temporarily store one or more of the data points.

15. The method of claim 13, wherein one or more of the motion sensors are coupled to the portable component and are adapted to be powered by the electrical energy.

100

PORTABLE HOUSING **102**

| MEMORY **112** | WIRELESS **116** | ELECTRICAL GENERATOR **104** |

LOGIC **110**

114

ENERGY STORAGE **106**

| CLOCK **120** | SENSOR(S) **108** | OUTPUT **118** |

**Fig. 1**

Fig. 2

EP 3 357 475 A1

**Fig. 3**

Start CPR → 30 compressions at 100 min⁻¹ → 2 ventilations → ... → 30 compressions at 100 min⁻¹ → 2 ventilations → 30 compressions at 100 min⁻¹ → Is the heart beating? → Stop CPR

No

2 min.

10 s

```
                              ┌─────────────┐
                              │    START    │
                              └──────┬──────┘
                                     │
                                     ▼
┌──────────────────────────────────────────────────────────────────┐
│   PLACE PORTABLE COMPONENT ON OUTER SURFACE OF PATIENT'S BODY      │
│                              402                                   │
└──────────────────────────────────┬───────────────────────────────┘
                                    │
                                    ▼
┌──────────────────────────────────────────────────────────────────┐
│   APPLY CHEST COMPRESSIONS TO CAUSE CORRESPONDING MOVEMENT OF      │
│                      PORTABLE COMPONENT                            │
│                              404                                   │
└──────────────────────────────────┬───────────────────────────────┘
                                    │
                                    ▼
┌──────────────────────────────────────────────────────────────────┐
│  CONVERT MOTION OF/FORCE IMPARTED ON PORTABLE COMPONENT INTO       │
│                      ELECTRICAL ENERGY                             │
│                              406                                   │
└──────────────────────────────────┬───────────────────────────────┘
                                    │
                                    ▼
┌──────────────────────────────────────────────────────────────────┐
│   USE HARVESTED ELECTRICAL ENERGY TO POWER ONE OR MORE SENSORS     │
│                              408                                   │
└──────────────────────────────────┬───────────────────────────────┘
                                    │
                                    ▼
┌──────────────────────────────────────────────────────────────────┐
│         DETECT ASPECT(S) OF MOTION OF PORTABLE COMPONENT           │
│                              410                                   │
└──────────────┬───────────────────────────────────┬───────────────┘
               │                                   │
               ▼                                   ▼
┌───────────────────────┐           ┌───────────────────────┐
│   USE HARVESTED        │           │   USE HARVESTED        │
│ ELECTRICAL ENERGY TO   │           │ ELECTRICAL ENERGY TO   │
│ STORE/TRANSMIT DATA    │           │   RENDER OUTPUT        │
│      POINT(S)          │           │        414             │
│        412             │           │                        │
└───────────────────────┘           └───────────────────────┘
```

**Fig. 4**

400

Europäisches
Patentamt

European
Patent Office

Office européen
des brevets

# EUROPEAN SEARCH REPORT

**Application Number**

EP 17 15 4735

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/043762 A1 (ECHT DEBRA S [US] ET AL) 24 February 2005 (2005-02-24) | 1,2,5, 9-15 | INV. A61H31/00 |
| Y | * paragraphs [0063] - [0103]; figures * | 4,6-8 | A61N1/39 A61B5/00 |
| X | WO 01/70332 A2 (CPRX LLC [US]) 27 September 2001 (2001-09-27) * pages 12-23; figures 1-6 * | 1,2,5, 9-11 | A61B5/0205 A61B5/021 A61B5/024 G06F19/00 |
| X | WO 2011/100507 A1 (ZOLL MEDICAL CORP [US]; TAN QING [US]; FREEMAN GARY A [US]; GEHEB FRED) 18 August 2011 (2011-08-18) * page 8, lines 14-31 - page 20, lines 4-32 * * pages 21-24; figures * | 1-3,5, 10,11 | |
| X | WO 2014/102725 A1 (KONINKL PHILIPS NV [NL]) 3 July 2014 (2014-07-03) * pages 3-8; figures * | 1-3,10, 11 | |
| Y | US 2016/128626 A1 (JOHNSON GUY R [US] ET AL) 12 May 2016 (2016-05-12) | 4,6-8 | |
| A | * paragraphs [0063] - [0131]; figures * | 1-3,5, 10-12 | TECHNICAL FIELDS SEARCHED (IPC) A61H A61B A61N G06F |

The present search report has been drawn up for all claims

| Place of search | Date of completion of the search | Examiner |
|---|---|---|
| Munich | 8 August 2017 | Teissier, Sara |

EPO FORM 1503 03.82 (P04C01)

## ANNEX TO THE EUROPEAN SEARCH REPORT
## ON EUROPEAN PATENT APPLICATION NO.

EP 17 15 4735

This annex lists the patent family members relating to the patent documents cited in the above-mentioned European search report.
The members are as contained in the European Patent Office EDP file on
The European Patent Office is in no way liable for these particulars which are merely given for the purpose of information.

08-08-2017

| Patent document cited in search report | | Publication date | Patent family member(s) | | Publication date |
|---|---|---|---|---|---|
| US 2005043762 | A1 | 24-02-2005 | US 2005043762 A1 | | 24-02-2005 |
| | | | US 2007123939 A1 | | 31-05-2007 |
| WO 0170332 | A2 | 27-09-2001 | AU 4585201 A | | 03-10-2001 |
| | | | US 6463327 B1 | | 08-10-2002 |
| | | | US 2002188332 A1 | | 12-12-2002 |
| | | | WO 0170332 A2 | | 27-09-2001 |
| WO 2011100507 | A1 | 18-08-2011 | CA 2789282 A1 | | 18-08-2011 |
| | | | CN 102781513 A | | 14-11-2012 |
| | | | EP 2533856 A1 | | 19-12-2012 |
| | | | JP 2013519446 A | | 30-05-2013 |
| | | | KR 20130044207 A | | 02-05-2013 |
| | | | US 2011202101 A1 | | 18-08-2011 |
| | | | WO 2011100507 A1 | | 18-08-2011 |
| WO 2014102725 | A1 | 03-07-2014 | CN 105307618 A | | 03-02-2016 |
| | | | EP 2938314 A1 | | 04-11-2015 |
| | | | JP 2016501664 A | | 21-01-2016 |
| | | | US 2015328083 A1 | | 19-11-2015 |
| | | | WO 2014102725 A1 | | 03-07-2014 |
| US 2016128626 | A1 | 12-05-2016 | NONE | | |

EPO FORM P0459

For more details about this annex : see Official Journal of the European Patent Office, No. 12/82